# EUROPÄISCHE PATENTSCHRIFT

(11) **EP 1 412 310 B1**
(45) Veröffentlichungstag und Bekanntmachung des Hinweises auf die Patenterteilung: **25.02.2009**
(21) Anmeldenummer: 02764727.0
(22) Anmeldetag: 18.07.2002
(51) Int. Cl.: C07C 5/10, C07C 5/327, C07C 13/18, C07C 15/02, C07C 11/06

(54) **VERFAHREN ZUR HYDRIERUNG VON AROMATISCHEN VERBINDUNGEN MIT RESTGAS ENTHALTENDEM WASSERSTOFF**
METHOD FOR THE HYDROGENATION OF AROMATIC COMPOUNDS WITH HYDROGEN CONTAINING RESIDUAL GAS
PROCEDE D'HYDROGENATION DE COMPOSES AROMATIQUES AU MOYEN D'HYDROGENE COMPRENANT DES GAZ RESIDUAIRES

(30) Priorität: 20.07.2001 DE 10135490
(43) Veröffentlichungstag der Anmeldung: 28.04.2004
(73) Patentinhaber: BASF SE, 67056 Ludwigshafen (DE)
(72) Erfinder: BÖTTCHER, Arnd, 67227 Frankenthal (DE); SCHWAB, Ekkehard, 67434 Neustadt (DE); KÄSTNER, Ralf, 55232 Alzey (DE); HENKELMANN, Jochem, 68165 Mannheim (DE); KAIBEL, Gerd, 68623 Lampertheim (DE); LAIB, Heinrich, 67117 Limburgerhof (DE)
(74) Vertreter: Isenbruck, Günter
(86) Internationale Anmeldenummer: PCT/EP2002/008023
(87) Internationale Veröffentlichungsnummer: WO 2003/010119

(56) Entgegenhaltungen:
- GB-A- 1 039 381
- US-A- 3 054 833
- US-A- 3 202 723
- US-A- 3 917 540
- US-A- 4 067 915
- US-B1- 6 255 530

## Beschreibung

Die vorliegende Erfindung betrifft ein Verfahren zur Hydrierung von substituierten oder unsubstituierten, insbesondere alkylsubstituierten ein- oder mehrkernigen Aromaten zu den entsprechenden Cycloaliphaten, insbesondere von Benzol zu Cyclohexan durch Inkontaktbringen des Aromaten mit einem Wasserstoff enthaltenden Gas in Gegenwart eines Katalysators, wobei Restgase enthaltender Wasserstoff eingesetzt wird.

Es existieren zahlreiche Verfahren zur Hydrierung von Benzol zu Cyclohexan. Diese Hydrierungen werden überwiegend an Nickel- und Platinkatalysatoren in der Gas- oder Flüssigphase durchgeführt (s. u.a. US 3 597 489 bzw US 2 898 387 bzw. GB 799 396). Typischerweise wird dabei zunächst in einem Hauptreaktor der größte Teil des Benzols zu Cyclohexan hydriert und anschließend in einem oder mehreren Nachreaktoren die Umsetzung von Cyclohexan komplettiert.

Die stark exotherme Hydrierreaktion erfordert eine sorgfältige Temperatur- und Verweilzeitkontrolle, um vollständigen Umsatz bei hoher Selektivität zu erreichen. Insbesondere muß eine signifikante Bildung von Methylcyclopentan unterdrückt werden, die bevorzugt bei höheren Temperaturen abläuft. Typische Cyclohexan-Spezifikationen fordern einen Benzol-Restgehalt < 100 ppm und einen Methylcyclopentan-Gehalt < 200 ppm. Auch der Gehalt an n-Paraffnen (n-Hexan, n-Pentan u. a.) ist kritisch. Diese unerwünschten Verbindungen entstehen ebenfalls bevorzugt bei höheren Hydriertemperaturen und lassen sich ebenso wie Methylcyclopentan nur durch aufwendige Trennoperationen (durch Extraktion, Rektifikation oder, wie in GB 1 341 057 beschrieben, Verwendung von Molekularsieben) vom produzierten Cyclohexan abtrennen. Auch der zur Hydrierung eingesetzte Katalysator hat einen starken Einfluß auf das Ausmaß der unerwünschten Methylcyclohexan-Bildung.

Vor diesem Hintergrund ist es erstrebenswert, die Hydrierung bei möglichst geringen Temperaturen durchzuführen. Dies ist jedoch dadurch limitiert, daß in Abhängigkeit von der Art des verwendeten Hydrierkatalysators erst ab höheren Temperaturen eine genügend hohe Hydrieraktivität des Katalysators erreicht wird, die ausreicht, wirtschaftliche Raum-Zeit-Ausbeuten zu erhalten.

Die für die Benzolhydrierung eingesetzten Nickel- und Platinkatalysatoren weisen eine Reihe von Nachteilen auf. Nickelkatalysatoren sind sehr empfindlich gegenüber schwefelhaltigen Verunreinigungen im Benzol, so daß man entweder sehr reines Benzol zur Hydrierung einsetzen muß, oder wie in GB 1 104 275 beschrieben, im Hauptreaktor einen Platinkatalysator einsetzt, der einen höheren Schwefelgehalt toleriert und so den Nachreaktor, der mit einem Nickelkatalysator gefüllt ist, schützt Eine andere Möglichkeit besteht in der Dotierung des Katalysators mit Rhenium (GB 1 155 539) oder in der Herstellung des Katalysators unter Verwendung von Ionenaustauschern (GB 1 144 499). Die Herstellung derartiger Katalysatoren ist jedoch aufwendig und teuer. Auch an Raney-Nickel kann die Hydrierung durchgeführt werden (US 3 202 723); von Nachteil ist jedoch die leichte Brennbarkeit dieses Katalysators. Auch homogene Nickelkatalysatoren können zur Hydrierung eingesetzt werden (EP-A 0 668 257). Diese Katalysatoren sind jedoch sehr wasserempfindlich, so daß das eingesetzte Benzol vor der Hydrierung zunächst in einer Trocknungskolonne auf einen Restwassergehalt < 1 ppm getrocknet werden muß. Ein weiterer Nachteil des Homogen-Katalysators ist, daß dieser nicht regeneriert werden kann.

In der WO 01/10802 wird ein Verfahren zur Herstellung von Cyclohexan durch Hydrierung von Benzol beschrieben, wobei die Wasserstoffquelle Verunreinigungen enthalten kann. Der Katalysator, der für das dort beschriebene Verfahren eingesetzt wird, umfaßt Nickel und Kupfer und optional weitere Metalle. Nachteilig an den dort verwendeten Nickel- und Kupferkatalysatoren ist jedoch, daß sie vor der eigentlichen Hydrierung vorreduziert werden müssen.

Platinkatalysatoren weisen weniger Nachteile als Nickelkatalysatoren auf, sind jedoch viel teurer in ihrer Herstellung. Sowohl bei der Verwendung von Platin- als auch von Nickelkatalysatoren sind sehr hohe Hydriertemperaturen notwendig, was zu einer signifikanten Bildung unerwünschter Nebenprodukte führen kann.

In der Patentliteratur finden sich auch Hinweise auf die Verwendung von rutheniumhaltiger Katalysatoren für diese Anwendung:

In der SU 319582 werden Ru-Suspensionskatalysatoren, die mit Pd, Pt oder Rh dotiert sind, zur Herstellung von Cyclohexan aus Benzol eingesetzt. Die Katalysatoren sind jedoch durch die Verwendung von Pd, Pt oder Rh sehr teuer. Ferner ist bei Suspensionskatalysatoren die Aufarbeitung und Wiedergewinung des Katalysators aufwendig und teuer.

In der SU 403658 wird ein mit Cr dotierter Ru-Katalysator zur Herstellung von Cyclohexan eingesetzt. Die Hydrierung erfolgt jedoch bei 180°C; hierbei wird eine signifikante Menge unerwünschter Nebenprodukte generiert.

In der US 3 917 540 werden Al₂O₃-geträgerte Katalysatoren zur Herstellung von Cyclohexan beansprucht. Diese enthalten als Aktivmetall ein Edelmetall aus der VIII. Nebengruppe des Periodensystems, weiterhin ein Alkalimetall sowie Technetium oder Rhenium. Die Hydrierung von Benzol läßt sich an derartigen Katalysatoren jedoch lediglich mit einer Selektivität von 99,5 % durchführen.

In der US 3 244 644 werden schließlich auf η-Al₂O₃ geträgerte Ruthenium-Hydrierkatalysatoren beschrieben, die sich auch zur Hydrierung von Benzol eignen sollen. Die Katalysatoren enthalten jedoch mindestens 5 % Aktivmetall; die Herstellung von η-Al₂O₃ ist aufwendig und teuer.

In der DE 100 50 711.5 wird ein Verfahren zur Hydrierung mittels Reaktivdestillatson in einer Reaktionskolonne unter Führung der Reaktionspartner im Gegenstrom über den in der Reaktionskolonne fixierten Katalysator beschrieben. In der PCT/EP 00/03326 wird ein Verfahren zur Hydrierung substituierter oder unsubstituierter Aromaten in Gegenwart eines Katalysators beschrieben, wobei der Katalysator als Aktivmetall mindestens ein Metall der VIII. Nebengruppe des Periodensystems aufgebracht auf einem Träger mit Makroporen umfaßt. Ein Verfahren zur Hydrierung von Aromaten in Gegenwart eines Katalysators mit einem Aktivmetall der VIII. Nebengruppe des Periodensystems auf einem monolithischen Träger wird in der DE 100 50 709.3 beschrieben. Die DE 101282 42.7 betrifft ein Verfahren zur Hydrierung organischer Verbindungen, insbesondere auch aromatischer Verbindungen, wobei der Katalysator als Aktivmetall Ruthenium auf einem Siliciumdioxid-Träger umfaßt. Zur Hydrierung wird bei den in diesen Anmeldungen erwähnten Verfahren ein Wasserstoff enthaltendes Gas mit dem Aromaten in Kontakt gebracht, wobei beliebige Gase verwendet werden können, die freien Wasserstoff enthalten und keine schädlichen Mengen an Katalysatorgiften. Insbesondere wird reiner Wasserstoff als Hydriergas verwendet

Der vorliegenden Erfindung lag daher die primäre Aufgabe zugrunde, ein Verfahren zur Hydrierung von substituierten oder unsubstituierten ein- oder mehrkernigen Aromaten zu den entsprechenden Cycloaliphaten, insbesondere von Benzol unter Erhalt von Cyclohexan, bereitzustellen, das es ermöglicht, den Cycloaliphaten mit sehr hoher Selektivität und Raum-Zeit-Ausbeute aus kostengünstigen Edukten zu erhalten.

Erfindungsgemäß wird diese Aufgabe gelöst durch ein Verfahren zur Hydrierung mindestens eines substituierten oder unsubstituierten ein- oder mehrkernigen Aromaten durch Inkontaktbringen des mindestens einen Aromaten mit einem Wasserstoff enthaltenden Gas in Gegenwart eines Katalysators, der als Aktivmetall mindestens ein Metall der VIII. Nebengruppe des Periodensystems umfaßt, wobei Restgase enthaltender Wasserstoff verwendet wird der bei der Dehydrierung von Ethylbenzol zu Styrol oder von Propan zu Propen anfällt, wobei der Restgase enthaltende Wasserstoff die folgende Zusammensetzung hat: 50 bis 98 Vol.-% H₂, < 5 Vol.-% CO, 0,01 bis 15 Vol.-% CO₂, 0 bis 2 Vol.-% Wasser, 0 bis 5 Vol.-% N₂ und 0 bis 5 Vol.-% sonstige aromatische oder aliphatische Kohlenwasserstoffe.

Durch die Verwendung von Restgas enthaltendem Wasserstoff kann bei dem erfindungsgemäßen Verfahren ein Reinigungsschritt der Edukte, der zu höheren Kosten führt, eingespart werden. Erfindungsgemäß ist es beispielsweise auch möglich, daß Restgase enthaltender Wasserstoff eingesetzt wird, der bei einem anderen Verfahren als Nebenprodukt anfällt. Damit kann das erfindungsgemäße Verfahren besonders kostengünstig durchgeführt werden.

Unter Restgasen werden im Rahmen der vorliegenden Erfindung beispielsweise Kohlenstoffmonoxid, Kohlenstoffdioxid, Wasser, Methan oder C2- bis C7-Kohlenwasserstoffe verstanden. Dabei enthält der erfindungsgemäß eingesetzte Restgas enthaltende Wasserstoff auch Gemische von zwei oder mehr davon. Neben den genannten Restgasen können beispielsweise auch geringe Mengen an Sauerstoff, Ethan, Benzol, Toluol, Ethylbenzol oder Styrol oder auch flüchtige oder leichtsiedende schwefelhaltige Verbindungen, wie Schwefelwasserstoff oder Thiophene im Restgas enthaltenden Wasserstoff enthalten sein.

Erfindungsgemäß hat der als Hydriergas eingesetzte Restgas enthaltende Wasserstoff die folgende Zusammensetzung: 50 bis 98 Vol.-% H₂, < 5 Vol.-% CO, 0,01 bis 15 Vol.-% CO₂, 0 bis 2 Vol.-% Wasser, 0 bis 5 Vol.-% N₂, O bis 5 Vol.-% sonstige aromatische oder aliphatische Kohlenwasserstoffe, bevorzugt >75 Vol.-% N₂, 0.01 bis 5 Vol.-% CO, 0,2 bis 10 Vol.-% CO₂, 0 bis 1 Vol.-% Wasser, 0 bis 5 Vol.-% N₂, 0 bis 3 Vol.-% sonstige aromatische oder aliphatische Kohlenwasserstoffe, insbesondere mit 3 bis 7 C-Atomen. Die Vol.-% Angaben sind auf die Summe der Bestandteile des Restgase enthaltenden Wasserstoffs bezogen. Neben den genannten können weitere Bestandteile, wie beispielsweise Ethan, Benzol, Ethylbenzol oder Styrol enthalten sein, insbesondere, wenn Restgas enthaltender Wasserstoff eingesetzt wird, der bei der Dehydrierung von Styrol anfäll.

Erfindungsgemäß geeigneter Restgase enthaltender Wasserstoff für die Hydrierung fällt bei der technisch durchgeführten Dehydrierung von Ethylbenzol zu Styrol oder von Propan zu Propen an.

Daher betrifft die Erfindung ein Verfahren, bei dem Restgase enthaltender Wasserstoff eingesetzt wird, der bei der Dehydrierung von Ethylbenzol zu Styrol oder von Propan zu Propen anfällt.

Bei der Dehydrierung von Propan zu Propen kann es sich beispielsweise um ein in Katalytica Stud. 4192 OD "Oxidative Dehydrogenation and alternative Dehydrogenation Processes" von Trifirò, Cavani beschriebenes Verfahren handeln.

Diese Verfahrensführung ist besonders vorteilhaft, da bei der Dehydrierung Restgase enthaltender Wasserstoff in großen Mengen anfällt, der so weiter genutzt werden kann, ohne daß kostenaufwendige Reinigungsschritte nötig sind. Das Wiedereinsetzten eines

Nebenprodukts der technischen Styrol- oder Propenherstellung führt zu einem besonders effektiven Verfahren, wobei gleichzeitig durch die Weiterverwertung eines Nebenprodukts die Umweltbelastung minimiert wird.

Es wurde überraschenderweise gefunden, daß sich die erfindungsgemäß im Wasserstoff enthaltenden Restgase nicht auf die Selektivität und Aktivität des Katalysators auswirken. Die substituierten oder unsubstituierten Aromaten können bei niedrigen Temperaturen selektiv und mit hoher Raum-Zeit Ausbeute zu den entsprechenden Cycloaliphaten hydriert werden. Auch die Produktqualität leidet nicht. Bei den erfindungsgemäß verwendbaren niedrigen Temperaturen unterbleibt die Bildung von unerwünschten Nebenprodukten, wie z.B. Methylcyclopentan oder anderen n-Paraffnen nahezu vollständig, so daß eine aufwendige Aufreinigung des produzierten Cycloaliphaten unnötig wird.

Der für das erfindungsgemäße Verfahren eingesetzte Katalysator umfaßt als Aktivmetall mindestens ein Metall der VIII. Nebengruppe des Periodensystems. Dabei kann der Katalysator als Aktivmetall beispielsweise ein Metall der VIII. Nebengruppe des Periodensystems alleine oder zusammen mit mindestens einem Metall der I., VII. oder VIII. Nebengruppe des Periodensystems umfassen. Bevorzugt ist das Aktivmetall mindestens ein Metall ausgewählt aus der Gruppe bestehend aus Ruthenium, Palladium und Rhodium. Besonders bevorzugt ist als Aktivmetall Ruthenium.

Das Aktivmetall kann erfindungsgemäß auf einen Träger aufgebracht sein. In einer Ausführungsform wird für das erfindungsgemäße Verfahren ein Katalysator eingesetzt, der als Aktivmetall mindestens ein Metall der VIII. Nebengruppe des Periodensystems, aufgebracht auf einen Makroporen aufweisenden Träger, umfaßt.

Daher betrifft die vorliegende Erfindung in einer Ausführungsform ein Verfahren zur Hydrierung mindestens eines substituierten oder unsubstituierten ein oder mehrkernigen Aromaten, wobei der Katalysator als Aktivmetall mindestens ein Metall der VIII. Nebengruppe des Periodensystems, aufgebracht auf einen Makroporen aufweisenden Träger, umfaßt.

Als Träger können prinzipiell alle Träger eingesetzt werden, die Makroporen aufweisen, d.h. Träger, die ausschließlich Makroporen aufweisen sowie solche, die neben Makroporen auch Meso- und/oder Mikroporen enthalten.

Als Aktivmetall können Metalle der VIII. Nebengruppe des Periodensystems alleine oder zusammen mit einem Metall der I., VII. oder VIII. Nebengruppe des Periodensystem, bevorzugt Ruthenium, Palladium oder Rhodium oder Gemische aus zwei oder mehr davon eingesetzt werden, wobei insbesondere Ruthenium als Aktivmetall verwendet wird. Unter den ebenfalls verwendbaren Metallen der I., VII. oder VIII. Nebengruppe des Periodensystems, die ebenfalls allesamt prinzipiell verwendbar sind, werden vorzugsweise Kupfer und/oder Rhenium eingesetzt.

Die Begriffe *"Makroporen"* und *"Mesoporen"* werden im Rahmen der vorliegenden Erfindung so verwendet, wie sie in Pure Appl. Chem., 45, S. 79 (1976*)* definiert sind, nämlich als Poren, deren Durchmesser oberhalb von 50 nm (Makroporen) oder deren Durchmesser zwischen 2 nm und 50 nm liegt (Mesoporen). *"Mikroporen "* sind ebenfalls entsprechend der obigen Literatur definiert und bezeichnen Poren mit einem Durchmesser von < 2 nm.

In einer bevorzugten Ausführungsform betrifft die vorliegende Erfindung ein derartiges Verfahren, wobei der Katalysator als Aktivmetall mindestens ein Metall der VIII. Nebengruppe des Periodensystems alleine oder zusammen mit mindestens einem Metall der I., VII. oder VIII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.-%, bezogen auf das Gesamtgewicht des Katalysators, beträgt (Katalysator 1). Weiter bevorzugt beträgt der mittlere Porendurchmesser des Trägers in diesem Katalysator mindestens 0,1 µm und die BET-Oberfläche höchstens 15 m²/g (Katalysator 1a).

Ferner betrifft sie ein derartiges Verfahren, wobei der Katalysator als Aktivmetall mindestens ein Metall der VIII. Nebengruppe des Periodensystems alleine oder zusammen mit mindestens einem Metall der I., VII. oder VIII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert (Katalysator 2).

Der Gehalt des Aktivmetalls beträgt im allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei den im folgenden beschriebenen, vorzugsweise eingesetzten Katalysatoren 1 bis 2 vorzugsweise verwendeten Gehalte nochmals bei der Diskussion dieser Katalysatoren einzeln angegeben sind.

Im folgenden sollen nunmehr die vorzugsweise verwendeten Katalysatoren 1 und 2 detailliert beschrieben werden. Dabei erfolgt die Beschreibung beispielhaft unter Bezugnahme auf die Verwendung von Ruthenium als Aktivmetall. Die untenstehenden Angaben sind auch auf die anderen verwendbaren Aktivmetalle, wie hierin definiert, übertragbar.

### Katalysator 1

Die erfindungsgemäß verwendeten Katalysatoren 1 können technisch hergestellt werden durch Auftragen mindestens eines Metalls der VIII. Nebengruppe des Periodensystems und gegebenenfalls mindestens eines Metalls der I., VII. oder VIII. Nebengruppe des Periodensystems auf einem geeigneten Träger.

Die Auftragung kann durch Tränken des Trägers in wäßrigen Metallsalzlösungen, wie z.B. wäßrigen Rutheniumsalzlösungen, durch Aufsprühen entsprechender Metallsalzlösungen auf den Träger oder durch andere geeignete Verfahren erreicht werden. Als Metallsalze der I., VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw getränkten Träger werden anschließend, vorzugsweise bei Temperaturen von 100 bis 150°C, getrocknet und wahlweise bei Temperaturen von 200 bis 600°C, vorzugsweise von 350 bis 450 °C calciniert. Bei getrennter Auftränkung wird der Katalysator nach jedem Tränkschritt getrocknet und wahlweise calciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen von ungefähr 30 bis ungefähr 600°C, vorzugsweise von ungefähr 150 bis ungefähr 450°C aktiviert. Vorzugsweise besteht der Gasstrom aus 50 bis 100 Vol.% H₂ und 0 bis 50 Vol.% N₂.

Die Metallsalzlösung oder -lösungen werden in einer solchen Menge auf den oder die Träger aufgebracht, daß der Gesamtgehalt an Aktivmetall, jeweils bezogen auf das Gesamtgewicht des Katalysators, ungefähr 0,01 bis ungefähr 30 Gew.-%, vorzugsweise ungefähr 0,01 bis ungefähr 5 Gew.-%, weiter bevorzugt ungefähr 0,01 bis ungefähr 1 Gew.-%, und insbesondere ungefähr 0,05 bis ungefähr 1 Gew.-% beträgt.

Die Metalloberfläche auf dem Katalysator 1 beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. Lemaitre et al. in "Characterization of Heterogeneous Catalysts", Hrsg. Francis Delanney, Marcel Dekker, New York 1984, S. 310 - 324, beschriebenen Chemisorptionsverfahren bestimmt.

Im erfindungsgemäß verwendeten Katalysator 1 beträgt das Verhältnis der Oberflächen des/der Aktivmetalls/-metalle und des Katalysatorträgers vorzugsweise weniger als ungefähr 0,05, wobei der untere Grenzwert bei ungefähr 0,0005 liegt.

Die zur Herstellung der erfindungsgemäß verwendeten Katalysatoren verwendbaren Trägermaterialien sind solche, die makroporös sind und einen mittleren Porendurchmesser von mindestens ungefähr 50 nm, vorzugsweise mindestens ungefähr 100 nm, insbesondere mindestens ungefähr 500 nm aufweisen und deren Oberfläche nach BET bei höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt. Der mittlere Porendurchmesser des Trägers beträgt vorzugsweise ungefähr 100 nm bis ungefähr 200 µm, weiter bevorzugt ungefähr 500 nm bis ungefähr 50 µm. Die Oberfläche nach BET des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine erfindungsgemäß bevorzugte Ausführungsform darstellt. -

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 196 24 484.6 zu entnehmen, deren diesbezüglicher Inhalt durch Bezugnahme vollständig in die vorliegende Anmeldung einbezogen wird.

Die zur Herstellung der erfindungsgemäß verwendeten Katalysatoren 1a, der eine bevorzugte Ausführungsform des Katalysators 1 darstellt, verwendbaren Trägermaterialien sind solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g aufweisen. Bevorzugt liegt der mittlere Porendurchmesser des dort verwendeten Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers. Auch dieser Katalysator weist bzgl. der Porendurchmesserverteilung die bereits oben beschriebene Bimodalität mit den analogen Verteilungen und das entsprechende bevorzugte Porenvolumen auf Weitere Details bezüglich Katalysator 1a sind der DE-A 196 04 791.9 zu entnehmen, deren diesbezüglicher Inhalt durch Bezugnahme vollständig in die vorliegende Anmeldung einbezogen wird.

### Katalysator 2

Die erfindungsgemäß verwendeten Katalysatoren 2 enthalten ein oder mehrere Metalle der VIII. Nebengruppe des Periodensystems als Aktivkomponente(n) auf einem Träger.

Die erfindungsgemäß verwendeten Katalysatoren 2 können technisch hergestellt werden durch Auftragen mindestens eines Aktivmetalls der VIII. Nebengruppe des Periodensystems, vorzugsweise Ruthenium, und gegebenenfalls mindestens eines Metalls der I., VII. oder VIII. Nebengruppe des Periodensystems auf einem geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen von 100 bis 150°C bevorzugt sind. Wahlweise können diese Träger bei Temperaturen von 200 bis 600°C, vorzugsweise von 350 bis 450°C calciniert werden. Anschließend werden die beschichteten Träger durch Behandlung in einem Gasstrom, der freien Wasserstoff enthält, bei Temperaturen von 30 bis 600°C, vorzugsweise von 100 bis 450°C und insbesondere von 100 bis 300°C aktiviert. Der Gasstrom besteht vorzugsweise aus 50 bis 100 Vol.% H₂ und 0 bis 50 Vol.% N₂.

Werden auf die Träger mehrere Aktivmetalle aufgetragen und erfolgt das Auftragen nacheinander, so kann der Träger nach jedem Auftragen bzw. Tränken bei Temperaturen von 100 bis 150°C getrocknet werden und wahlweise bei Temperaturen von 200 bis 600°C calciniert werden. Dabei kann die Reihenfolge, in der die Metallsalzlösung aufgetragen oder aufgetränkt wird, beliebig gewählt werden.

Die Metallsalzlösung wird in einer solchen Menge auf den/die Träger aufgebracht, daß der Gehalt an Aktivmetall 0,01 bis 30 Ges.-%, vorzugsweise 0,01 bis 10 Ges.-%, weiter bevorzugt 0,01 bis 5 Gew %, und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemiscrptionsverfahren gemessen, wie es in J. Lemaitre et al., "Characterization of Heterogeneous Catalysts", Hrsg. Francis Delanney, Marcel Dekker, New York (1984), S. 310 - 324, beschrieben ist

Im erfindungsgemäß verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß verwendbaren Träger eine Porenverteilung auf, dergemäß ungefähr 5 bis ungefähr 50%, vorzugsweise ungefähr 10 bis ungefähr 45%, weiter bevorzugt ungefähr 10 bis ungefähr 30 und insbesondere ungefähr 15 bis ungefähr 25% des Porenvolumens von Makroporen mit Porendurchmessern im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Anteile der Porenvolumina zu 100% addiert.

Das Gesamtporenvolumen der erfindungsgemäß verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g des Trägers.

Die Oberfläche des Trägers wird nach dem BET Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysator 2 sind der DE-A 196 24 485.4 zu entnehmen, deren diesbezüglicher Inhalt durch Bezugnahme vollständig in die vorliegenden Anmeldung einbezogen wird.

Erfindungsgemäß ist es auch möglich, daß die Hydrierung in Gegenwart eines Katalysators in einer Reaktionskolonne unter Führung der Reaktionspartner über einen in der Reaktionskolonne fixierten Katalysator durchgeführt wird.

Demgemäß betrifft die vorliegende Erfindung in einer weiteren Ausführungsform ein Verfahren zur Hydrierung mindestens eines substituierten oder unsubstituierten ein- oder mehrkernigen Aromaten, wobei die Hydrierung in einer Reaktionskolonne unter Führung der Reaktionspartner über den in der Reaktionskolonne fixierten Katalysator durchgeführt wird.

Vorzugsweise werden die Reaktionspartner im Gegenstrom über den(die) in der Reaktionskolonne fixierten Katalysator(en) geführt.

Sofern die als Produkt erwünschten Cycloaliphaten über einen Seitenabzug entnommen werden, werden die leichter siedenden Komponenten (Leichtsieder) am Kopf der Kolonne abgezogen. Entsprechend werden die höher als der Cycloaliphat siedenden Komponenten (Hochsieder) am Sumpf der Kolonne erhalten. Demgemäß wird die Fahrweise an die jeweiligen Nebenprodukte, die in Aromaten vorhanden sind oder während der Reaktion entstehen, angepaßt. Beispielsweise werden Leichtsieder über Kopf abgezogen und entsprechend hochsiedenden Komponenten über Sumpf, während der Cycloaliphat über einen Seitenabzug erhalten wird.

Falls keine hochsiedenden Nebenprodukte oder Nebenkomponenten anfallen, wird das Wertprodukt über Sumpf abgezogen. Selbstverständlich ist erfindungsgemäß auch eine Fahrweise möglich, in der die Cycloaliphaten als Wertprodukte über den Seitenabzug und im Sumpf der Kolonne anfallen.

Dabei wird erfindungsgemäß über das Rücklaufverhältnis in der Kolonne und/oder dem Energieeintrag in die Kolonne gesteuert, ob die Cycloaliphaten am Seitenabzug oder im Sumpf der Kolonne anfallen. Am Seitenabzug wird das Produkt bevorzugt in flüssiger Form entnommen.

Im Rahmen dieser Ausführungsform wird die katalytische Hydrierung in einer Reaktionskolonne an einem heterogenen Katalysator durchgeführt, wobei prinzipiell alle für diese Anwendung geeigneten Katalysatoren eingesetzt werden können, insbesondere die zuvor näher beschriebenen Katalysatoren 1, 1 a und 2.

Ferner können die folgenden weiteren Heterogenkatalysatoren mit Aktivmetallen eingesetzt werden. Als Aktivmetalle können prinzipiell alle Metalle der VIII. Nebengruppe des Periodensystems eingesetzt werden. Vorzugsweise werden für diese Ausführungsform als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird.

Unter den ebenfalls verwendbaren Metallen der I. und VII. oder aber der I. und der VIII. Nebengruppe des Periodensystems, die ebenfalls allesamt prinzipiell verwendbar sind, werden vorzugsweise Kupfer und/oder Rhenium eingesetzt.

Besonders bevorzugt wird Ruthenium allein eingesetzt Ein Vorteil der Verwendung des Hydriermetalls Ruthenium ist, daß hierdurch im Vergleich zu den deutlich teureren Hydriermetallen Platin, Palladium oder Rhodium erhebliche Kosten bei der Katalysatorherstellung eingespart werden können.

Der in dem erfindungsgemäßen Verfahren bevorzugt eingesetzte Ruthenium-Katalysator ist entweder in Form einer Schüttung oder als katalytisch aktive Destillationspackung oder in Kombination von beiden in der Kolonne plaziert Die Form einer derartigen Schüttung oder Destillationspackung ist dem Fachmann aus dem Stand der Technik bereits bekannt.

Beispiele für metallische Werkstoffe als Trägermaterialien sind Reinmetalle wie Eisen, Kupfer, Nickel, Silber, Aluminium Zirkonium, Tantal und Titan oder Legierungen wie Stähle oder Edelstähle, wie z.B. Nickel-, Chrom- und/oder Molybdän-Stahl. Ferner können Messing, Phosphorbronze, Monell und/oder Neusilber oder Kombinationen aus zwei oder mehr der oben genannten Materialien eingesetzt werden.

Beispiele für keramische Werkstoffe sind Aluminiumoxid (Al₂O₃), Siliciumdioxid (SiO₂) Zirkoniumdioxid (ZrO₂) Cordierit und/oder Steatit

Beispiele für synthetische Trägermaterialien sind beispielsweise Kunststoffe wie Polyamide, Polyester, Polyether, Polyvinyle, Polyolefine wie Polyethylen, Polypropylen, Polytetrafluorethylen, Polyketone, Polyetherketone, Polyethersulfone, Epoxidharze, Aldehydharze, Harnstoff- und/oder Melamin-Aldehydharze. Weiterhin können Kohlenstoff oder Glasfasern als Träger eingesetzt werden.

Bevorzugterweise werden strukturierte Träger in Form von Metallgeweben, -gestricken, -gewirken, -blechen oder -filzen, Kohlefasergeweben oder -filzen oder Kunststoffgeweben oder -gestricken verwendet. Als Drahtgewebe kommen, Gewebe aus webbaren Metalldrähten wie Eisen, Federstahl, Messing, Phosphorbronze, Reinnickel, Monel, Aluminium, Silber, Neusilber, Nickel, Chromnickel, Chromstahl, nicht rostenden, säurebeständigen und hochhitzebeständigen Chromnickelstählen sowie Titan in Betracht.

Ebenfalls können Gewebe aus anorganischen Materialien verwendet werden, wie beispielsweise Gewebe aus keramischen Werkstoffen wie Al₂O₃ und/oder SiO₂.

Die strukturierten Träger oder Monolithe können aus metallischen, anorganischen, organischen oder synthetischen Materialien oder Kombinationen solcher Materialien bestehen.

Auch synthetische Drähte und Gewebe aus Kunststoffen sind gemäß einer Ausführungsform der Erfindung einsetzbar.

Monolithe aus Gewebepackungen sind besonders bevorzugt, da sie hohe Querschnittsbelastungen von Gas und Flüssigkeit aushalten und dabei einen nur unwesentlichen Abrieb zeigen.

In einer weiteren besonders bevorzugten Ausführungsform werden metallische, strukturierte Träger oder Monolithen verwendet, die aus Edelstahl bestehen, der vorzugsweise beim Tempern unter Luft und anschließendem Abkühlen eine Aufrauung der Oberfläche zeigt. Diese Eigenschaften zeigen insbesondere Edelstähle, bei denen sich oberhalb einer spezifischen Entmischungstemperatur ein Legierungsbestandteil an der Oberfläche anreichert und in Gegenwart von Sauerstoff durch Oxidation eine festhaftende rauhe oxidische Oberflächenschicht bildet. Ein solcher Legierungsbestandteil kann beispielsweise aus Aluminium oder Chrom sein, aus denen sich entsprechend eine Oberflächenschicht aus Al₂O₃ oder Cr₂O₃ bildet. Beispiele für Edelstähle sind die mit den Werkstoff-Nummern 1.4767, 1.4401, 1.4301, 2.4610, 1.4765, 1.4847 und 1.4571. Diese Stähle können vorzugsweise durch Tempern an der Luft bei 400 bis 1.100 °C über einen Zeitraum von 1 Stunde bis zu 20 Stunden und anschließendes Abkühlenlassen auf Raumtemperatur thermisch aufgerauht werden.

In einer bevorzugten Ausführungsform ist der heterogene Katalysator ein mit Ruthenium beschichtetes Gewebe, das gleichzeitig als Destillationspackung wirkt.

In einer noch weiter bevorzugten Ausführungsform des erfindungsgemäßen Verfahrens besteht die Destillationspackung aus mit Ruthenium beschichteten Metallfäden, wobei besonders bevorzugt die Edelstähle der Nummer 1.4301 oder 1.4767 eingesetzt werden.

Weitere Details bezüglich dieser Ausführungsform, insbesondere zur Herstellung des Katalysators, sind in der DE 100 50 711.5 beschrieben, deren diesbezüglicher Inhalt durch Bezugnahme vollständig in die vorliegende Anmeldung einbezogen wird.

In einer weiteren Ausführungsform betrifft die vorliegende Erfindung ein Verfahren zur Hydrierung eines substituierten oder unsubstituierten ein- oder mehrkernigen Aromaten, wobei der Katalysator als Aktivmetall ein Metall der VIII. Nebengruppe, aufgebracht auf einen strukturierten oder monolithischen Träger, umfaßt.

Unter strukturierten Trägern werden im Zusammenhang mit der vorliegenden Erfindung solche Träger verstanden, die eine regelmäßige flächige oder räumliche Struktur aufweisen und sich dadurch von Trägern in Partikelform, die als loses Haufwerk verwendet werden, unterscheiden. Beispiele für strukturierte Träger sind aus Fäden oder Drähten aufgebaute Träger, meist in Form von Trägerbahnen, wie Gewebe, Gestricke, Gewirke oder Filze. Strukturierte Träger können auch Folien oder Bleche sein, die auch Aussparungen aufweisen können, wie Lochbleche oder Streckmetalle. Solche im wesentlichen flächigen strukturierten Träger können zu dem Einsatzzweck zu entsprechend geformten räumlichen Strukturen, sogenannten Monolithen bzw. monolithischen Trägem, geformt werden, die ihrerseits beispielsweise als Katalysatorpackungen oder Kolonnenpackungen verwendet werden können. Packungen können aus mehreren Monolithen bestehen. Es ist ebenfalls möglich, Monolithe nicht aus flächigen Trägerbahnen aufzubauen, sondern sie ohne Zwischenstufen direkt herzustellen, beispielsweise die dem Fachmann bekannten keramischen Monolithe mit Strömungskanälen.

Vor Aufbringung der Aktivmetalle und gegebenenfalls Promotoren können die strukturierten oder monolithischen Träger gegebenenfalls mit einem, zwei oder mehreren Oxiden beschichtet werden. Dies kann physikalisch, beispielsweise durch Besputtern, erfolgen. Hierbei werden in oxidierender Atmosphäre in einer dünnen Schicht Oxide, wie z.B. Al₂O₃ auf den Träger aufgebracht.

Die strukturierten Träger können entweder vor oder nach der Aufbringung der Aktivmetalle bzw. Promotoren beispielsweise mittels einer Zahnradwelle verformt bzw. zusammengerollt werden, um ein monolithisches Katalysatorelement zu erhalten.

Die Katalysatoren, die im Rahmen der vorliegenden Erfindung eingesetzt werden, können auch Promotoren zur Dotierung des Katalysators enthalten, beispielsweise Alkali- und/oder Erdalkalimetalle, wie Lithium, Natrium, Kalium, Rubidium, Cäsium, Magnesium, Calcium, Strontium und Barium; Silicium, Kohlenstoff, Titan, Zirkonium, Wolfram, sowie die Lanthanoiden und Actinoiden; Münzmetalle wie Kupfer, Silber und/oder Gold, Zink, Zinn, Bismuth, Antimon, Molybdän, Wolfram und/oder andere Promotoren wie Schwefel und/oder Selen.

Die erfindungsgemäß verwendeten Katalysatoren können technisch hergestellt werden durch Auftragen mindestens eines Metalls der VIII. Nebengruppe des Periodensystems und gegebenenfalls mindestens eines Promotors auf einen der vorstehend beschriebenen Träger.

Die Aufbringung der Aktivmetalle und gegebenenfalls Promotoren auf den vorstehend beschriebenen Trägern kann erfolgen, indem man die Aktivmetalle im Vakuum verdampft und kontinuierlich auf den Träger kondensiert. Eine andere Möglichkeit besteht darin, die Aktivmetalle durch Tränken mit Lösungen, welche die Aktivmetalle und gegebenenfalls Promotoren enthalten, auf den Trägern aufzubringen. Eine weitere Möglichkeit besteht darin, die Aktivmetalle und gegebenenfalls Promotoren durch chemische Methoden, wie der Chemical Vapour Deposition (CVD) auf den Trägern aufzubringen.

Eine weitere Möglichkeit besteht darin, die fixierten Träger, insbesondere Netze, Gewebe und Gestricke in-situ mit der Tränklösung zu beaufschlagen. Details bezüglich dieser Vorgehensweise sind der EP-A 0 803 488 zu entnehmen.

Die so hergestellten Katalysatoren können direkt eingesetzt werden oder vor ihrem Einsatz getempert und/oder calciniert werden, und können sowohl vorreduziert als auch im nichtreduzierten Zustand eingesetzt werden.

Wahlweise wird der Träger vor der Aufbringung der Aktivmetalle und gegebenenfalls Promotoren vorbehandelt. Eine Vorbehandlung ist beispielsweise vorteilhaft, wenn die Haftung der Aktivkomponenten auf dem Träger verbessert werden soll. Beispiele für eine Vorbehandlung sind die Beschichtung des Trägers mit Haftvermittlern oder eine Aufrauhung mit mechanischen (etwa Schleifen, Sandstrahlen) oder thermischen Verfahren wie Erhitzen, in der Regel an Luft, Plasmaätzen oder Glimmen.

Im folgenden sollen nunmehr die vorzugsweise verwendeten Katalysatoren 3 und 4 beschrieben werden; bezüglich allgemeiner Merkmale der Katalysatoren 3 und 4 wird auf die vorstehenden Ausführungen verwiesen.

### Katalysator 3

Bevorzugt wird der strukturierte Träger oder Monolith, der für Katalysator 3 eingesetzt wird, vorbehandelt, beispielsweise durch vorstehend beschriebenes Tempern an der Luft (thermisches Aufrauhen) und anschließende Abkühlung. Daraufhin wird der Träger vorzugsweise mit einer das Aktivmetall enthaltenden Lösung (Tränkmedium) getränkt. Nachfolgend kann, wenn es sich um einen im wesentlichen flächigen strukturierten Träger handelt, dieser zu einem monolithischen Katalysatorelement verarbeitet werden.

Wenn der Träger metallisch ist, beispielsweise aus Edelstahl besteht, wird dieser vorzugsweise durch Tempern an der Luft bei 400 bis 1.100°C für einen Zeitraum von 1 Stunde bis 20 Stunden und anschließendem Abkühlen auf Raumtemperatur thermisch aufgerauht.

Das Tränken des Trägers mit der Lösung kann durch Eintauchen, Spülen oder Besprühen erfolgen.

Das Tränkmedium weist vorzugsweise eine Oberflächenspannung von höchstens 50 mN/m auf. In weiter bevorzugter Form hat das Tränkmedium eine Oberflächenspannung von höchstens 40 mN/m. Der Minimalwert der Oberflächenspannung ist im allgemeinen frei wählbar. In bevorzugter Form hat das Tränkmedium jedoch eine Oberflächenspannung von mindestens 10 mN/m und in besonders bevorzugter Form von mindestens 25 mN/m. Die Oberflächenspannung wird nach der dem Fachmann bekannten OECD-Ring-Methode (ISO 304, vgl. Amtsblatt der EG Nr. L 383 vom 29.12.1992, Seiten A/47 - A/53) gemessen.

Das Tränkmedium enthält vorzugsweise ein Lösungs- und/oder Suspensionsmittel, beispielsweise Wasser, in welchem die Aktivmetalle vorzugsweise in Form ihrer Metallsalze gelöst sind.

Das Tränkmedium kann wahlweise auch Promotoren zur Dotierung des Katalysators enthalten. In diesem Zusammenhang wird auf vorstehende allgemeine Ausführungen verwiesen.

Ein im Tränkmedium enthaltenes Lösungs- und/oder Suspensionsmittel wird so gewählt, daß die aufzubringenden Aktivkomponenten, Aktivmetalle, Promotoren oder ihre Vorläufer darin und/oder damit keine unerwünschten Reaktionen zeigen.

Als Lösungs- und/oder Suspensionsmittel können die bekannten und technisch gängigen Lösungsmittel verwendet werden, beispielsweise aromatische oder aliphatische Kohlenwasserstoffe wie Benzol, Toluol, Xylol, Cumol, Pentan, Hexan, Heptan, Kohlenwasserstoffschnitte wie Benzin, Ligroin, Weißöl, Alkohole, Diole, Polyole wie Methanol, Ethanol, die beiden Propanolisomeren, die vier Butanolisomeren, Glykol, Glycerin, Ether wie Diethylether, Di-n-butylether, Methyl-tert.-butylether, Ethyl-tert.-butylether, Methyl-tert.-amylether, Ethyl-tert.-amylether, Diphenylether, Ethylenglykoldimethylether, Diethylenglykoldimethylether, Triethylenglykoldimethylether, oder Wasser. Die verwendeten organischen Lösungs- oder Suspensionsmittel können auch substituiert sein, beispielsweise mit Halogenen, wie Chlorbenzol, oder mit Nitrogruppen, wie Nitrobenzol. Die Lösungs- oder Suspensionsmittel werden einzeln oder im Gemisch verwendet.

Das Tränkmedium kann darüber hinaus, falls nötig, Hilfsstoffe enthalten. Beispielsweise enthält das Tränkmedium sauer oder basisch reagierende Verbindungen oder Puffer, wenn dies zur Stabilisierung oder Löslichkeit mindestens einer ihrer Aktivkomponenten oder deren Vorläufer erforderlich oder vorteilhaft ist.

Bevorzugt werden lösliche Salz der Aktivkomponenten in einem Lösungsmittel vollständig gelöst. Vorteilhafterweise wird eine wäßrige Lösung von Aktivkomponenten verwendet.

Wenn die Aktivmasse aus Metallen besteht, wird in besonders bevorzugter Weise entweder eine wäßrige, salpetersaure Lösung der Nitrate der Metalle oder eine wäßrige, ammoniakalische Lösung von Aminkomplexen der Metalle verwendet. Wenn die Aktivkomponenten aus amorphen Metalloxiden bestehen, wird in bevorzugter Weise ein wäßriges Sol des Oxids, das wahlweise stabilisiert ist, verwendet

Die Oberflächenspannung des Tränkmediums kann durch geeignete oberflächenaktive Stoffe wie anionische oder nichtanionische Tenside eingestellt werden. Der getränkte Träger wird im Regelfall nach der Tränkung bei Temperaturen von 100 bis etwa 120 °C getrocknet und wahlweise bei Temperaturen von 120 bis 650 °C, vorzugsweise von 120 bis 400 °C calciniert.

Ein im wesentlichen flächig strukturierter Träger kann nach der thermischen Behandlung zu einem dem Einsatzzweck entsprechend geformten räumlichen Gebilde verformt werden. Die Verformung kann beispielsweise durch Arbeitsschritte wie Zuschneiden, Wellen der Bahnen, Anordnen oder Fixieren der gewellten Bahnen in Form eines Monolithen mit parallelen oder kreuzweisen Kanälen erfolgen. Die Verformung zum Monolithen wird wahlweise vor der Tränkung, der Trocknung, der thermischen Behandlung oder der chemischen Behandlung durchgeführt.

Weitere Details bezüglich Katalysator 3 und seiner Herstellung sind der DE-A 198 27 385.1 zu entnehmen, deren diesbezüglicher Inhalt durch Bezugnahme vollständig in die vorliegende Anmeldung einbezogen wird.

### Katalysator 4

Der strukturierte Träger oder Monolith, der für Katalysator 4 verwendet wird, wird vorzugsweise vorbehandelt, beispielsweise durch Tempern an der Luft und anschließendes Abkühlen. Nachfolgend wird der Träger bevorzugt mit mindestens einem Aktivmetall im Vakuum beschichtet. Anschließend kann, wenn es sich um einen im wesentlichen flächig ausgebildeten strukturierten Träger handelt, dieser zu einem monolithischen Katalysatorelement verarbeitet werden.

Vorzugsweise werden neben dem/den Aktivmetall/en im Vakuum auch Promotoren zur Dotierung des Katalysators auf das Trägermaterial aufgebracht. Bezüglich möglicher Promotoren wird auf die vorstehenden allgemeinen Ausführungen verwiesen.

Das Trägermaterial besteht vorzugsweise aus Metall, besonders bevorzugt aus Edelstahl, weite bevorzugt Edelstähle der hierin weiter vorne genannten Nummern. Die Vorbehandlung des Trägers erfolgt bevorzugt durch Tempern des Metallträgers bei Temperaturen von 600 bis 1.100 °C, vorzugsweise bei 800 bis 1.000 °C, 1 bis 20, vorzugsweise 1 bis 10 Stunden an der Luft. Anschließend wird der Träger abgekühlt.

Die aktiven Komponenten (Aktivmetalle und Promotoren) können durch Aufdampfen und Aufsputtern auf den Träger aufgebracht werden. Dazu wird der Träger im Vakuum bei einem Druck von 10⁻³ bis 10⁻⁸ mbar, vorzugsweise mittels einer Verdampfungseinrichtung, z.B. Elektronenstrahlverdampfung oder Sputtereinrichtung mit den Aktivkomponenten gleichzeitig oder nacheinander in diskontinuierlicher oder kontinuierlicher Fahrweise beschichtet. Zur Formierung des Katalysators kann eine Temperung unter Inertgas oder Luft nachgeschaltet werden.

Das Aufbringen der Aktivkomponenten kann in mehreren Schichten erfolgen. Der so erhaltene Katalysator kann zu einem Monolithen weiterverarbeitet werden. Diesbezüglich wird u.a. auf die Ausführungen zu Katalysator 3 verwiesen. Vorzugsweise wird der Katalysator verarbeitet, indem das Katalysatorgewebe bzw. die Katalysatorfolie mittels einer Zahnradwalze verformt wird (gewellt, gesickt) und glattes und gewelltes Gewebe zu einem zylinderförmigen Monolithen mit gleichartigen senkrechten Kanälen zusammengerollt wird.

Weitere Details bezüglich Katalysator 4 und seiner Herstellung sind der EP 0 564 830 zu entnehmen, deren diesbezüglicher Inhalt durch Bezugnahme vollständig in die vorliegende Anmeldung einbezogen wird.

### Katalysator 5

In einer weiteren bevorzugten Ausführungsform betrifft die vorliegende Erfindung ein Verfahren zur Hydrierung mindestens eines substituierten oder unsubstituierten ein- oder mehrkernigen Aromaten, wobei der Katalysator als Aktivmetall Ruthenium alleine oder zusammen mit mindestens einem weiteren Metall der I., VII. oder VIII. Nebenguppe des Periodensystems enthält, und erhältlich ist durch:
i) ein oder mehrfaches Behandeln eines Trägermaterials auf Basis von amorphem Siliziumdioxid mit einer halogenfreien wässrigen Lösung einer niedermolekularen Rutheniumverbindung und anschließendes Trocknen des behandelten Trägermaterials bei einer Temperatur unterhalb 200°C,
ii) Reduktion des in i) erhaltenen Feststoffs mit Wasserstoff bei einer Temperatur im Bereich von 100 bis 350°C,
wobei man und Schritt ii) unmittelbar im Anschluss an Schritt i) durchführt.

Derartige Katalysatoren (Katalysator 5) weisen eine besonderes gute Verteilung des Rutheniums auf der Oberfläche des Trägermaterials auf. Herstellungsbedingt liegt das Ruthenium in den erfindungsgemäß eingesetzten Katalysatoren als metallisches Ruthenium vor. Das Ruthenium liegt auf dem Trägermaterial in atomar-dipserser Form und/oder in Form von Ruthenium-Partikeln vor, die nahezu ausschließlich, d.h. zu mehr als 90 %, vorzugsweise zu mehr als 95 %, bezogen auf die Anzahl der sichtbaren Partikel, als isolierte Partikel mit Durchmessern unterhalb 10 nm, insbesondere unterhalb 7 nm vorliegen. Mit anderen Worten, der Katalysator enthält im Wesentlichen keine, d.h. zu weniger als 10 %, insbesondere weniger als 5 % Ruthenium-Partikel und/oder Agglomerate von Rutheniumpartikeln mit Durchmessern oberhalb 10 nm. Durch die Verwendung halogenfreier Rutheniumprekursoren und Lösungsmittel bei der Herstellung liegt der Chlorgehalt der erfindungsgemäßen Katalysatoren zudem unterhalb 0,05 Gew.-%, bezogen auf das Gesamtgewicht des Katalysators.

Ein wesentlicher Aspekt dieses Katalysators (Kat. 5) ist die Verwendung eines Trägermaterials auf der Basis von amorphem Siliziumdioxid. Unter dem Begriff "amorph" versteht man in diesem Zusammenhang, dass der Anteil kristalliner Siliziumdioxid-Phasen weniger als 10 % des Trägermaterials ausmacht. Die zur Herstellung der erfindungsgemäß eingesetzten Katalysators verwendeten Trägermaterialien können allerdings Überstrukturen aufweisen, die durch regelmässige Anordnung von Poren im Trägermaterial gebildet werden.

Als Trägermaterialien kommen grundsätzliche alle amorphen Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.% des Trägermaterials auch ein anderes oxidisches Material sein können, z.B. MgO, CaO, TiO₂, ZrO₂, Fe₂O₃ oder Alkalimetalloxid. Es versteht sich von selbst, das das eingesetzte Trägermaterial ebenfalls halogenfrei ist, das heißt, der Halogen-Gehalt beträgt weniger als 500 ppm. Vorzugsweise enthält das Trägermaterial nicht mehr als 1 Gew.-% und insbesondere nicht mehr als 0,5 Gew.-% und insbesondere keine nachweisbaren Mengen an Aluminiumoxid, gerechnet als Al₂O₃. In einer bevorzugten Ausführungsform verwendet man Trägermaterialien, die weniger als 500 ppm Fe₂O₃ enthalten. Der Anteil an Alkalimetalloxid resultiert in der Regel aus der Herstellung des Trägermaterials und kann bis zu 2 Gew.-% betragen. Häufig beträgt er weniger als 1 Gew.-%. Geeignet sind auch Alkalimetalloxid-freie Träger (< 0,1 Gew.-%). Der Anteil an MgO, CaO, TiO₂ bzw. an ZrO₂ kann bis zu 10 Gew.-% des Trägermaterials ausmachen und beträgt vorzugsweise nicht mehr als 5 Gew.-%. Geeignet sind aber auch Trägermaterialien, die keine nachweisbaren Mengen dieser Metalloxide enthalten (< 0,1 Gew.-%).

Bevorzugt sind Trägermaterialien, die eine spezifische Oberfläche im Bereich von 50 bis 700 m²/g, insbesondere im Bereich von 80 bis 600 m²/g und speziell im Bereich von 100 bis 600 m²/g aufweisen (BET-Oberfläche nach DIN 66131). Pulverförmige Trägermaterialien weisen vorzugsweise eine spezifische Oberfläche im Bereich von 200 bis 600 m²/g auf, und Formkörper vorzugsweise vorzugsweise eine spezifische Oberfläche im Bereich von 200 bis 300 m²/g.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 5th ed. on CD-ROM). Sie können sowohl natürlichen Ursprungs als auch künstlich hergestellt worden sein. Beispiele für geeignete amorphe Trägermaterialien auf Basis von Siliziumdioxid sind Kieselgur, Kieselgele, pyrogene Kieselsäure und Fällungskieselsäure. In einer bevorzugten Ausführungsform der Erfindung enthalten die Katalysatoren Kieselgele als Trägermaterialien.

Je nach Ausgestaltung des erfindungsgemäßen Hydrierverfahrens kann das Trägermaterial unterschiedliche Gestalt aufweisen. Sofern das Verfahren als Suspensionsverfahren ausgestaltet ist, wird zur Herstellung des Katalysators üblicherweise das Trägermaterial in Form eines feinteiligen Pulvers eingesetzt. Bei Einsatz des Katalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylinderm Sternen und dergleichen aufweisen können. Die Abmessungen dieser Formkörper bewegen sich üblicherweise im Bereich von 1 mm bis 25 mm. Häufig werden Katalysatorstränge mit Strangdurchmessern von 2 bis 5 mm und Stranglängen von 2 bis 25 mm eingesetzt.

In einer bevorzugten Ausführungsform betrifft die vorliegende Erfindung daher ein Verfahren zur Hydrierung mindestens eines substituierten oder unsubstituierten ein- oder mehrkernigen Aromaten, wobei der Katalysator weniger als 0,05 Gew.-% Halogen, bezogen auf das Gesamtgewicht des Katalysators, enthält und:
- ein Trägermaterial auf Basis von amorphem Siliziumdioxid und
- elementares Ruthenium alleine oder zusammen mit mindestens einem Metall der I., VII. oder VIII. Nebengruppe des Periodensystems als Metall, das auf dem Träger in atomar-disperser Form oder in Form von Metall-Partikeln oder in atomar-disperser Form und in Form von Metall-Partikeln vorliegt,
wobei der Katalysator im Wesentlichen keine Metall-Partikel oder Agglomerate mit Durchmessern oberhalb 10 nm aufweist.

In einer weiter bevorzugten Ausführungsform betrifft die vorliegende Erfindung ein Verfahren, wobei der Katalysator regeneriert wird.

Geeignete Verfahren zur Regenerierung eines derartigen Katalysators sind beispielsweise Behandeln mit halogenfreier Säure, wie in der US 4,072,628 beschrieben, Behandeln mit wäßrigem Wasserstoffperoxid oder anderen halogenfreien oxidierenden Substanzen oder Regenerierung mit Sauerstoff, wie beispielsweise in der BE 882 279 beschrieben.

Im Folgenden soll der erfindungsgemäß einsetzbare Katalysator 5 näher beschrieben werden:

Der Gehalt an Ruthenium des Katalysators 5 kann über einen breiten Bereich variiert werden. In der Regel wird er wenigstens 0,1 Gew.%, vorzugsweise wenigstens 0,2 Gew.-% betragen und häufig einen Wert von 10 Gew.-%, jeweils bezogen auf das Gewicht des Trägermaterials, nicht überschreiten. Vorzugsweise liegt der Gehalt an Ruthenium im Bereich von 0,2 bis 7 Gew.-% und insbesondere im Bereich von 0,4 bis 5 Gew.%.

Zur Herstellung der erfindungsgemäß eingesetzten Katalysators (5) wird das Trägermaterial zunächst mit einer halogenfreien wässrigen Lösung einer niedermolekularen Rutheniumverbindung, im Folgenden als (Ruthenium)prekursor bezeichnet, in einer Weise behandelt, dass die gewünschte Menge an Ruthenium vom Trägermaterial aufgenommen wird. Dieser Schritt wird im Folgenden auch als Tränken bezeichnet. Anschliessend wird der so behandelte Träger bei den oben angegebenen Temperaturen getrocknet. Gegebenenfalls wird dann der so erhaltene Feststoff erneut mit der wässrigen Lösung des Rutheniumprekursors behandelt und erneut getrocknet. Dieser Vorgang wird so oft wiederholt, bis die vom Trägermaterial aufgenommene Menge an Rutheniumverbindung dem gewünschten Rutheniumgehalt im Katalysator entspricht

Das Behandeln bzw. Tränken des Trägermaterials kann in unterschiedlicher Weise erfolgen und richtet sich in bekannter Weise nach der Gestalt des Trägermaterials. Beispielsweise kann man das Trägermaterial mit der Prekursor-Lösung besprühen oder spülen oder das Trägermaterial in der Prekursor-Lösung suspendieren. Beispielsweise kann man das Trägermaterial in der wässrigen Lösung des Rutheniumprekursors suspendieren und nach einer gewissen Zeit vom wässrigen Überstand abfiltrieren. Über die aufgenommene Flüssigkeitsmenge und die Ruthenium-Konzentration der Lösung kann dann der Rutheniumgehalt des Katalysators in einfacher Weise gesteuert werden. Das Tränken des Trägermaterials kann beispielsweise auch dadurch erfolgen, dass man den Träger mit einer definierten Menge der wässrigen Lösung des Rutheniumprekursors behandelt, die der maximalen Flüssigkeitsmenge entspricht, die das Trägermaterial aufnehmen kann. Zu diesem Zweck kann man beispielsweise das Trägermaterial mit der Flüssigkeitsmenge besprühen. Geeignete Apparaturen hierfür sind die zum Vermengen von Flüssigkeiten und Feststoffen üblicherweise verwendeten (siehe beispielsweise Vauck, Müller "Grundoperationen chemischer Verfabrenstechnik", 10. Auflage, Deutscher Verlag für die Kunststoffindustrie, Leipzig, 1994, S. 405ff.), beispielsweise geeignet sind Taumeltrockner, Tränktrommeln, Trommelmischer, Schaufelmischer und dergleichen. Monolithische Träger werden üblicherweise mit den wässrigen Lösungen des Rutheniumprekursors gespült.

Die zum Tränken eingesetzten wässrigen Lösungen sind erfindungsgemäß halogenfrei, d.h. sie enthalten kein oder weniger als 500 ppm Halogen. Als Rutheniumprekursoren werden daher nur solche Rutheniumverbindungen eingesetzt, die kein chemisch gebundenes Halogen enthalten und die in dem wässrigen Lösungsmittel hinreichend löslich sind. Hierzu zählen z.B. Ruthenium(III)nitrosylnitrat (Ru(NO)(NO₃)₃), Ruthenium(III)acetat sowie die Alkalimetallruthenate(IV) wie Natrium- und Kaliumruthenat(IV).

Der Begriff "wässrig" bezeichnet hier Wasser sowie Mischungen von Wasser mit bis zu 50 Vol.-%, vorzugsweise nicht mehr als 30 Vol.-% und insbesondere nicht mehr als 10 Vol.-% eines oder mehrerer mit Wasser mischbarer organischer Lösungsmittel, z.B. Mischungen von Wasser mit C₁-C₄-Alkanolen wie Methanol, Ethanol, n- oder Isopropanol. Häufig setzt man Wasser als alleiniges Lösungsmittel ein. Das wässrige Lösungsmittel wird häufig auch beispielsweise eine halogenfreie Säure, z.B. Salpetersäure, Schwefelsäure oder Essigsäure, zur Stabilisierung des Rutheniumprekursors in der Lösung enthalten. Die Konzentration des Rutheniumprekursors in den wässrigen Lösungen richtet sich naturgemäss nach der aufzubringenden Menge an Rutheniumprekursor und der Aufnahmekapazität des Trägermaterials für die wässrige Lösung und liegt in der Regel im Bereich von 0,1 bis 20 Gew.-%.

Das Trocknen kann nach den üblichen Verfahren der Feststofftrocknung unter Einhaltung der obengenannten Temperaturen erfolgen. Die Einhaltung der erfindungsgemäßen Obergrenze der Trocknungstemperaturen ist für die Qualität, d.h. die Aktivität des Katalysators wichtig. Ein Überschreiten der oben angegebenen Trocknungstemperaturen führt zu einem deutlichen Verlust an Aktivität. Ein Kalzinieren des Trägers bei höheren Temperaturen, z.B. oberhalb 300°C oder gar 400°C, wie es im Stand der Technik vorgeschlagen wird, ist nicht nur überflüssig sondern wirkt sich auch nachteilig auf die Aktivität des Katalysators aus.

Die Trocknung des in mit dem Rutheniumprekursors getränkten Feststoff erfolgt üblicherweise unter Normaldruck wobei zur Förderung der Trocknung auch ein verminderter Druck angewendet werden kann. Häufig wird man zur Förderung der Trocknung einen Gasstrom über bzw. durch das zu trocknende Gut leiten, z.B. Luft oder Stickstoff.

Die Trocknungsdauer hängt naturgemäß von dem gewünschten Grad der Trocknung und der Trocknungstemperatur ab und liegt in der Regel im Bereich von 2 h bis 30 h, vorzugsweise im Bereich von 4 bis 15 h.

Vorzugsweise wird die Trocknung des behandelten Trägermaterials soweit geführt, dass der Gehalt an Wasser bzw. an flüchtigen Lösungsmittelbestandteilen vor der Reduktion ii) weniger als 5 Gew.-% und insbesondere nicht mehr als 2 Gew.-%, besonders bevorzugt nicht mehr als 1 Gew.-%, bezogen auf das Gesamtgewicht des Feststoffs ausmacht. Die angegebenen Gewichtsanteile beziehen sich auf den Gewichtsverlust des Feststoffs, bestimmt bei einer Temperatur von 300°C einem Druck von 1 bar und einer Dauer von 10 min. Auf diese Weise kann die Aktivität der erfindungsgemäßen Katalysatoren weiter gesteigert werden.

Vorzugsweise erfolgt das Trocknen unter Bewegen des mit der Prekursor-Lösung behandelten Feststoffs, beispielsweise durch Trocknen des Feststoffs in einem Drehrohrofen oder einem Drehkugelofen. Auf diese Weise kann die Aktivität der erfindungsgemäßen Katalysatoren weiter gesteigert werden.

Die Überführung des nach dem Trocknen erhaltenen Feststoffs in seine katalytisch aktive Form erfolgt erfindungsgemäß durch Hydrieren des Feststoffs bei den oben angegebenen Temperaturen in an sich bekannter Weise.

Zu diesem Zweck bringt man das Trägermaterial bei den oben angegebenen Temperaturen mit Wasserstoff oder einer Mischung aus Wasserstoff und einem Inertgas in Kontakt. Der Wasserstoffpartialdruck ist für das Ergebnis der Reduktion von untergeordneter Bedeutung und kann im Bereich von 0,2 bar bis 1,5 bar variiert werden. Häufig erfolgt die Hydrierung des Katalysatormaterials bei Wasserstoffnormaldruck im Wasserstoffstrom. Vorzugsweise erfolgt das Hydrieren unter Bewegen des in i) erhaltenen Feststoffs, beispielsweise durch Hydrieren des Feststoffs in einem Drehrohrofen oder einem Drehkugelofen. Auf diese Weise kann die Aktivität der erfindungsgemäßen Katalysatoren weiter gesteigert werden.

Im Anschluss an die Hydrierung kann der Katalysator zur Verbesserung der Handhabbarkeit in bekannter Weise passiviert werden, z.B. indem man den Katalysator kurzfristig mit einem Sauerstoff-haltigen Gas, z.B. Luft, vorzugsweise jedoch mit einer 1 bis 10 Vol.% Sauerstoff enthaltenden Inertgasmischung behandelt.

Derartige Katalysatoren sind in der DE 101 282 42.7 oder der DE 101 282 05.2 beschrieben, deren diesbezüglicher Inhalt durch Bezugnahme vollständig in die vorliegende Anmeldung einbezogen wird.

Im Folgenden sollen weitere Details zu dem erfindungsgemäßen Verfahren näher beschrieben werden:

Innerhalb des erfindungsgemäßen Verfahrens können prinzipell alle substituierten oder unsubstituierten ein oder mehrkernigen Aromaten einzeln oder als Gemische aus zwei oder mehr davon, vorzugsweise einzeln, eingesetzt werden.

Sofern ein substituierter ein- oder mehrkerniger Aromat in das erfindungsgemäße Verfahren eingesetzt wird, kann der Substituent inert gegenüber einer Hydrierung sein. Es ist im Rahmen der vorliegenden Erfindung aber auch möglich, daß der Aromat Substituenten mit einer oder mehreren hydrierbaren Gruppen aufweist. Je nach Verfahrensführung- ist es erfindungsgemäß möglich, daß nur der Aromat oder der Aromat und die hydrierbare(n) Gruppe(n) hydriert werden. Hydrierbare Gruppen sind insbesondere C-C-, C-O-, N-O- oder C-N-Mehrfachbindungen. Beispielsweise können erfindungsgemäß ein- oder mehrkernige Aromaten hydriert werden, die mit einer oder mehreren C-C-Doppelbindungen oder Carbonylgruppen substituiert sind.

Im Rahmen der vorliegenden Erfindung kann der Aromat auch einen oder mehrere Alkylsubstituenten aufweisen. Die Länge der Alkylgruppen unterliegt dabei auch keinerlei besonderen Beschränkungen, im allgemeinen handelt es sich jedoch um C1- bis C30-, vorzugsweise C1- bis C18-, insbesondere C1- bis C4-Alkylgruppen. Im einzelnen sind als Edukte für das vorliegenden Verfahren insbesondere die folgenden Aromaten zu nennen:

Benzol, Toluol, Xylole, Cumol, Diphenylmethan, Tri-, Tetra-, Penta- und Hexabenzole, Triphenylmethan, alkylsubstituierte Naphthaline, Naphthalin, alkylsubstituierte Anthracene, Anthracen, alkylsubstituierte Tetraline und Tetralin.

Vorzugsweise werden erfindungsgemäß unsubstituierte oder alkylsubstituierte Aromaten hydriert. Besonders bevorzugt wird im Rahmen des vorliegenden Verfahrens Benzol zu Cyclohexan hydriert

Erfindungsgemäß können als Edukte beispielsweise aber auch Heteroaromaten oder Aromaten eingesetzt werden, die mindestens eine an den aromatischen Kern gebundene Hydroxyl- oder Aminogruppe aufweisen.

Im Rahmen der vorliegenden Erfindung werden unter Heteroaromaten aromatische Verbindungen verstanden, die mindestens ein Heteroatom, beispielsweise ein N-, S- oder O-Atom, im aromatischen Ring aufweisen.

Sofern der ein- oder mehrkernige Aromat substituiert ist, ist es erfindungsgemäß möglich, das cis/trans-Isomerenverhältnis der Hydrierungsprodukte in einem weiten Bereich zu variieren.

Im Rahmen des erfindungsgemäßen Verfahrens wird die Hydrierung im allgemeinen bei einer Temperatur von ungefähr 50 bis 250°C, vorzugsweise ungefähr 70 bis 200°C, ungefähr 80 bis 180°C, und insbesondere ungefähr 80 bis 150°C durchgeführt. Dabei können die niedrigsten Temperaturen bei Verwendung von Ruthenium als Aktivmetall gefahren werden. Die dabei verwendeten Drücke liegen in der Regel bei oberhalb von 1 bar, vorzugsweise ungefähr 1 bis ungefähr 200 bar, weiter bevorzugt ungefähr 10 bis ungefähr 50 bar.

Das erfindungsgemäße Verfahren kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge des zur Hydrierung vorgesehenen Aromaten ungefähr 0,05 bis ungefähr 3 kg pro Liter Katalysator pro Stunde, weiter bevorzugt ungefähr 0,2 bis ungefähr 2 kg pro Liter Katalysator pro Stunde.

Als Hydriergas wird Restgase enthaltender Wasserstoff eingesetzt. Dabei enthält das Hydriergas im Rahmen der vorliegenden Erfindung mehr als 75 % Wasserstoff bezogen auf die Summe von Wasserstoff und Restgas, insbesondere mehr als 80 %, besonders bevorzugt mehr als 90 %. Erfindungsgemäß kann Restgase enthaltender Wasserstoff eingesetzt werden, wie er bei der Dehydrierung von Ethylbenzol zu Styrol oder von Propan zu Propen anfällt.

Erfindungsgemäß ist es auch möglich, daß der Wasserstoff, der bei einer Dehydrierungsreaktion anfällt, einem Reinigungsschritt unterzogen wird, bei dem höhersiedende aromatische oder aliphatische Verbindungen oder Wasser oder höhersiedende aromatische oder aliphatische Verbindungen und Wasser abgetrennt werden. Unter "höhersiedenden aromatischen oder aliphatischen Verbindungen" werden dabei im Rahmen der vorliegenden Erfindung solche Verbindungen verstanden, deren Siedepunkt über dem von Kohlenstoffmonoxid und Kohlenstoffdioxid liegt. Kohlenstoffmonoxid und Kohlenstoffdioxid werden bei einem derartigen Reinigungsschritt nicht abgetrennt.

Die erfindungsgemäße Hydrierung kann in Ab- oder Anwesenheit eines Lösungs- oder Verdünnungsmittels durchgeführt werden, d.h. es ist nicht erforderlich, die Hydrierung in Lösung durchzuführen.

Als Lösungs- oder Verdünnungsmittel kann jedes geeignete Lösungsmittel- oder -Verdünnungsmittel eingesetzt werden. Die Auswahl ist dabei nicht kritisch, solange das eingesetzte Lösungs- oder Verdünnungsmittel in der Lage ist, mit dem zu hydrierenden Aromaten eine homogene Lösung zu bilden.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung des zur Hydrierung vorgesehenen Aromaten führen.

Bei Verwendung eines Lösungsmittels wird im Rahmen des erfindungsgemäßen Verfahrens das bei der Hydrierung gebildete Produkt, also der oder die jeweilige(n) Cycloaliphat(en) als Lösungsmittel eingesetzt, gegebenenfalls neben anderen Lösungs- oder Verdünnungsmitteln. In jedem Fall kann ein Teil des im Verfahren gebildeten Produkts dem noch zu hydrierenden Aromaten beigemischt werden. Bezogen auf das Gewicht des zur Hydrierung vorgesehenen Aromaten wird vorzugsweise die 1- bis 30fache, besonders bevorzugt die 5- bis 20fache, insbesondere die 5- bis 10fache Menge an Produkt als Lösungs- oder Verdünnungsmittel zugemischt Insbesondere betrifft die vorliegende Erfindung eine Hydrierung der hier in Rede stehenden Art, wobei Benzol in Gegenwart des hierin definierten Katalysators 2 bei einer Temperatur von 50 bis 180°C oder 80 bis 180°C, bevorzugt 80 bis 150°C, insbesondere 100 bis 120°C oder 110°C, zu Cyclohexan hydriert wird.

Die Erfindung betrifft in einer bevorzugten Ausführungsform ein Verfahren zur Hydrierung von Benzol, wobei Benzol bei einer Temperatur von 80 bis 180°C umgesetzt wird und als Aktivmetall Ruthenium alleine verwendet wird.

Die Erfindung soll anhand der folgenden Beispiele erläutert werden:

### Beispiele

### Beispiel 1: Hydrierung in der Flüssigphase

Der Dehydrierwasserstoff aus einer Styrol-Produktionsanlage mit einer Zusammensetzung von 90,6 % H₂, 0,01 % O₂, 0,52 % N₂, 0,82 % Methan, 0,18 % CO, 5,40 % CO₂, 0,22 % Ethylen, 0,04 % Ethan, 0,06 % Propan, 0,14 % Benzol, 343 ppm Toluol, 976 ppm Ethylbenzol und 625 ppm Styrol wurde zweistufig von ca. 1 bar auf 20 bar komprimiert und dann direkt zur Hydrierung verwendet. 0,5 kg Ruthenium-Katalysator wurden in einen Rohrreaktor eingefüllt. Der Katalysator (0,5 % Ruthenium auf Al₂O₃-Kugeln) wurde wie in der PCT/EP00/03326 beschrieben hergestellt. Anschließend wurde ohne vorhergehende Aktivierung bei 20 bar und 150°C mit der Hydrierung von Benzol begonnen. Die Hydrierung wurde kontinuierlich in Rieselfahrweise ausgeführt, wobei ein Teil des Hydrierungsaustrags über eine Umlaufpumpe zurückgeführt und dem Einsatzstoff vor dem Reaktor zugemischt wurde. Bezogen auf die Menge an Benzol wurde so die zehnfache Menge an Hydrierungsprodukt als Lösungsmittel zugesetzt. Die kontinuierlich dem Reaktor zugeführte Benzolmenge entsprach einer Kalalysatorbelastung von 0,5 kg/(l * h).

Bei der Hydrierung wurde Benzol vollständig umgesetzt. Cyclohexan wurde mit einer Selektivität von 99,9 % gebildet. Methylcyclopentan wurde nicht nachgewiesen.

### Beispiel 2: Hydrierung in der Gasphase

100 ml Ruthenium-Katalysator wurden in einen ölbeheizten Durchflußreaktor (Glas) eingefüllt. Der Katalysator (0,5 % Ruthenium auf Al₂O₃-Kugeln) wurde wie in der PCT/EP00/03326 beschrieben hergestellt Anschließend wurde ohne vorhergehende Aktivierung unter Normaldruck mit einer Hydrierung von Benzol begonnen. Dabei wurde Benzol über einen Vorverdampfer (80°C) in die Gasphase gebracht und zusammen mit Wasserstoff, der mit einer Zusammensetzung von 90,6 % H₂, 0,01 % O₂, 0,52 % N₂, 0,82 % Methan, 0,18 % CO, 5,40 % CO₂, 0,22 % Ethylen, 0,04 % Ethan, 0,06 % Propan, 0,14 % Benzol, 343 ppm Toluol, 976 ppm Ethylbenzol und 625 ppm Styrol direkt aus der Styrolsynthese erhalten wurde, bei 100°C und einer Belastung von 0,3 kg/(l*/h) kontinuierlich im geraden Durchgang durch das Katalysatorbett geleitet Der Austrag wurde in einer Kühlfalle kondensiert.

Dabei konnte Benzol vollständig zu Cyclohexan hydriert werden. Cyclohexan wurde mit einer Selektivität von 99,9 % gebildet. Methylcyclcpentan wurde nicht nachgewiesen.

## Patentansprüche

1. Verfahren zur Hydrierung mindestens eines substituierten oder unsubstituierten ein- oder mehrkernigen Aromaten durch Inkontaktbringen des mindestens einen Aromaten mit einem Wasserstoff enthaltenden Gas in Gegenwart eines Katalysators, der als Aktivmetall mindestens ein Metall der VIII. Nebengruppe des Periodensystems umfaßt, wobei Restgase enthaltender Wasserstoff verwendet wird, der bei der Dehydrierung von Ethylbenzol zu Styrol oder von Propan zu Propen anfällt, wobei der Restgase enthaltende Wasserstoff die folgende Zusammensetzung hat: 50 bis 98 Vol.-% H₂, 25 Vol.-% CO, 0,01 bis 15 Vol.-% CO₂, 0 bis 2 Vol.-% Wasser, 0 bis 5 Vol.-% N₂ und 0 bis 5 Vol.-% sonstige aromatische oder aliphatische Kohlenwasserstoffe.

2. Verfahren nach Anspruch 1, **dadurch gekennzeichnet, daß** der Restgase enthaltende Wasserstoff Kohlenstoffmonoxid, Kohlenstoffdioxid, Wasser, Methan oder C2- bis C7-Kohlenwasserstoffe oder Gemische von zwei oder mehr davon enthält.

3. Verfahren nach einem der Ansprüche 1 oder 2, **dadurch gekennzeichnet, daß** der Restgase enthaltende Wasserstoff mindestens 2 Vol,-% Restgase ausgewählt aus der Gruppe bestehend aus Kohlenstoffmonoxid Kohlenstoffdioxid, Wasser, Methan oder C2- bis C7-Kohlenwasserstoffe enthält.

4. Verfahren nach einem der Ansprüche 1 bis 3, **dadurch gekennzeichnet, daß** der Katalysator als Aktivmetall mindestens ein Metall der VII. Nebengruppe des Periodensystems, aufgebracht auf einem Träger mit Makroporen, umfaßt.

5. Verfahren nach einem der Ansprüche 1_ bis 3, **dadurch gekennzeichnet, daß** der Katalysator als Aktivmetall mindestens ein Metall der VIII. Nebengruppe des Periodensystems in einer Menge von 0,01, bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10,000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert

6. Verfahren nach einem der Ansprüche 1 bis 3, **dadurch gekennzeichnet, daß** der Katalysator als Aktivmetall mindestens ein Metall der VIII. Nebengruppe des Periodensystems, aufgebracht auf einen strukturierten oder monolithischen Träger, umfaßt.

7. Verfahren nach einem der vorherigen Ansprüche, **dadurch gekennzeichnet, daß** die Hydrierung in einer Reaktionskolonne, unter Führung der Reaktionspartner über einen in der Reaktionskolonne fixierten Katalysator durchgeführt wird.

8. Verfahren nach einem der Ansprüche 1 bis 3, **dadurch gekennzeichnet, daß** der Katalysator als Aktivmetall Ruthenium alleine oder zusammen mit mindestens einem weiteren Metall der I., VII. oder VIII. Nebengruppe des Periodensystems enthält, erhältlich durch:
i) ein oder mehrfaches Behandeln eines Trägermaterials auf Basis von amorphem Siliziumdioxid mit einer halogenfreien wässrigen Lösung einer niedermolekularen Rutheniumverbindung und anschließendes Trocknen des behandelten Trägermaterials bei einer Temperatur unterhalb 200°C,
ii) Reduktion des in i) erhaltenen Feststoffs mit Wasserstoff bei einer Temperatur im Bereich von 100 bis 350°C,
wobei man Schritt ii) unmittelbar im Anschluss an Schritt i) durchführt

9. Verfahren nach Anspruch 8, **dadurch gekennzeichnet, daß** der Katalysator weniger als 0,05 Gew.-% Halogen, bezogen auf das Gesamtgewicht des Katalysators, enthält und:
- ein Trägermaterial auf Basis von amorphem Siliziumdioxid und
- elementares Ruthenium alleine oder zusammen mit mindestens einem Metall der I., VII, oder VIII. Nebengruppe des Periodensystems als Metall, das auf dem Träger in atomar-disperser Form oder in Form von Metall-Partikeln oder in atomar-disperser Form und in Form von Metall-Partikeln vorliegt,
wobei der Katalysator im Wesentlichen keine Metall-Partikel oder Agglomerate mit Durchmessern oberhalb 10 nm aufweist

10. Verfahren nach einem der vorherigen Ansprüche, **dadurch gekennzeichnet, daß** der ein- oder mehrkernige Aromat ausgewählt wird unter Benzol, Toluol, Xylolen, Cumol, Diphenylmethan, Tri-, Tetra-, Penta- und Hexabenzolen, Triphenylmethan, akylsubstituierten Naphthalinen, Naphthalin, alkyl-substituierte Anthracenen, Anthracen, alkylsubstituierten Tetralinen und Tetralin.

11. Verfahren nach einem der vorherigen Ansprüche, **dadurch gekennzeichnet, daß** Benzol zu Cyclohexan umgesetzt wird.

12. Verfahren nach einem der vorherigen Ansprüche, **dadurch gekennzeichnet, daß** Benzol bei einer Temperatur von 80 bis 180°C umgesetzt wird und als Aktivmetall Ruthenium alleine verwendet wird.

## Claims

1. A process for the hydrogenation of at least one substituted or unsubstituted, monocyclic or polycyclic aromatic by bringing the aromatic or aromatics into contact with a hydrogen-comprising gas in the presence of a catalyst which comprises at least one metal of transition group VIII of the Periodic Table as active metal, wherein hydrogen in which residual gases are present is obtained in the dehydrogenation of ethylbenzene to styrene or of propane to propene, and which is used where the hydrogen in which residual gases are present has the following composition: 50 to 98% by volume of H₂, 25% by volume of CO, 0.01 to 15% by volume of CO₂ 0 to 2% by volume of water, 0 to 5% by volume of N₂ and 0 to 5% by volume of miscellaneous aromatic or aliphatic hydrocarbons.

2. The process according to claim 1, wherein the hydrogen in which residual gases are present comprises carbon monoxide, carbon dioxide, water, methane or C2-C7-hydrocarbons or mixtures of two or more thereof.

3. The process according to claim 1 or 2, wherein the hydrogen in which residual gases are present comprises at least 2% by volume of residual gases selected from the group consisting of carbon monoxide, carbon dioxide, water, methane and C2-C7-hydrocarbons.

4. The process according to any of claims 1 to 3, wherein the catalyst comprises at least one metal of transition group VIII of the Periodic Table as active metal applied to a macroporous support.

5. The process according to any of claims 1 to 3, wherein the catalyst comprises, as active metal, at least one metal of transition group VIII of the Periodic Table in an amount of from 0.01 to 30% by weight, based on the total weight of the catalyst, applied to a support, where from 10 to 50% of the pore volume of the support is made up by macropores having a pore diameter in the range - from 50 nm to 10,000 nm and from 50 to 90% of the pore volume of the support is made up by mesopores having a pore diameter in the range from 2 to 50 nm, where the sum of the pore volumes is 100%.

6. The process according to any of claims 1 to 3, wherein the catalyst comprises at least one metal of transition group VIII of the Periodic Table as active metal applied to a structured or monolithic support.

7. The process according to any of the preceding claims, wherein the hydrogenation is carried out in a reaction column with the reactants being passed over a catalyst fixed in the reaction column.

8. The process according to any of claims 1 to 3, wherein the catalyst comprises, as active metal, ruthenium alone or together with at least one further metal of transition group I, VII or VIII of the Periodic Table and is obtainable by:
i) single or multiple treatment of a support material based on amorphous silicon dioxide with a halogen-free aqueous solution of a low molecular weight ruthenium compound and a subsequent drying of the treated support material at a temperature below 200°C,
ii) reduction of the solid obtained in i) by means of hydrogen at a temperature in the range from 100 to 350°C,
where step ii) is carried out directly after step i) .

9. The process according to claim 8, wherein the catalyst comprises less than 0.05% by weight of halogen, based on the total weight of the catalyst, and:
- a support material based on amorphous silicon dioxide and
- elemental ruthenium either alone or together with at least one metal of transition group I, VII or VIII of the Periodic Table as metal which is present on the support in atomically dispersed form or in the form of metal particles or in atomically dispersed form and in the form of metal particles,
where the catalyst contains essentially no metal particles or agglomerates having diameters above 10 nm.

10. The process according to any of the preceding claims, wherein the monocyclic or polycyclic aromatic is selected from among benzene, toluene, xylenes, cumene, diphenylmethane, tribenzenes, tetrabenzenes, pentabenzenes and hexabenzenes, triphenylmethane, alkyl-substituted naphthalenes, naphthalene, alkyl-substituted anthracenes, anthracene, alkyl-substituted tetralins and tetralin.

11. The process according to any of the preceding claims, wherein benzene is converted into cyclohexane.

12. The process according to any of the preceding claims, wherein benzene is reacted at from 80 to 180°C and ruthenium alone is used as active metal.

## Revendications

1. Procédé pour l'hydrogénation d'au moins un aromatique substitué ou non substitué, à un ou plusieurs noyaux, par la mise en contact dudit au moins un aromatique avec un gaz contenant de l'hydrogène en présence d'un catalyseur qui comprend comme métal actif au moins un métal du VIIIème groupe secondaire du système périodique, où on utilise de l'hydrogène contenant des gaz résiduels, qui est produit lors de la déshydrogénation d'éthylènebenzène en styrène ou de propane en propène, l'hydrogène contenant des gaz résiduels présentant la composition suivante : 50 à 98% en volume de H₂, < 5% en volume de CO, 0,01 à 15% en volume de CO₂, 0 à 2% en volume d'eau, 0 à 5% en volume de N₂ et 0 à 5% en volume d'autres hydrocarbures aromatiques ou aliphatiques.

2. Procédé selon la revendication 1, **caractérisé en ce que** l'hydrogène contenant des gaz résiduels contient du monoxyde de carbone, du dioxyde de carbone, de l'eau, du méthane ou des hydrocarbures en C₂ à C₇ ou des mélanges de deux ou plus de ceux-ci.

3. Procédé selon l'une quelconque des revendications 1 ou 2, **caractérisé en ce que** l'hydrogène contenant des gaz résiduels contient au moins 2% en volume de gaz résiduels choisis dans le groupe constitué par le monoxyde de carbone, le dioxyde de carbone, l'eau, le méthane ou les hydrocarbures en C₂ à C₇.

4. Procédé selon l'une quelconque des revendications 1 à 3, **caractérisé en ce que** le catalyseur comprend comme métal actif au moins un métal du VIIIème groupe secondaire du système périodique, appliqué sur un support avec des macropores.

5. Procédé selon l'une quelconque des revendications 1 à 3, **caractérisé en ce que** le catalyseur contient comme métal actif au moins un métal du VIIIème groupe secondaire du système périodique en une quantité de 0,01 à 30% en poids, par rapport au poids total du catalyseur, appliqué sur un support, où 10 à 50% du volume des pores du support sont formés par des macropores présentant un diamètre des pores dans la plage de 50 nm à 10 000 nm et 50 à 90% du volume des pores du support sont formés par des mésopores présentant un diamètre des pores dans la plage de 2 à 50 nm, la somme des proportions des volumes des pores s'additionnant à 100%.

6. Procédé selon l'une quelconque des revendications 1 à 3, **caractérisé en ce que** le catalyseur comprend comme métal actif au moins un métal du VIIIème groupe secondaire du système périodique, appliqué sur un support structuré ou monolithique.

7. Procédé selon l'une quelconque des revendications précédentes, **caractérisé en ce que** l'hydrogénation est réalisée dans une colonne de réaction avec guidage des partenaires de réaction sur un catalyseur fixé dans la colonne de réaction.

8. Procédé selon l'une quelconque des revendications 1 à 3, **caractérisé en ce que** le catalyseur contient comme métal actif du ruthénium, seul ou ensemble avec au moins un autre métal du Ier, VIIème ou VIIIème groupe secondaire du système périodique, pouvant être obtenu par :
i) traitement simple ou multiple d'un matériau support à base de dioxyde de silicium amorphe avec une solution aqueuse exempte d'halogène d'un composé du ruthénium de bas poids moléculaire et séchage consécutif du matériau support traité à une température inférieure à 200°C,
ii) réduction du solide obtenu dans i) avec de l'hydrogène à une température dans la plage de 100 à 350°C,
l'étape ii) étant réalisée de manière directement consécutive à l'étape i).

9. Procédé selon la revendication 8, **caractérisé en ce que** le catalyseur contient moins de 0,05% en poids d'halogène, par rapport au poids total du catalyseur, et :
- un matériau support à base de dioxyde de silicium amorphe et
- du ruthénium élémentaire, seul ou ensemble avec au moins un métal du Ier, VIIème ou VIIIème groupe secondaire du système périodique comme métal, qui se trouve sur le support sous forme atomique dispersée ou sous forme de particules métalliques ou sous forme atomique dispersée et sous forme de particules métalliques,
le catalyseur ne présentant essentiellement pas de particules métalliques ni d'agglomérats présentant des diamètres supérieurs à 10 nm.

10. Procédé selon l'une quelconque des revendications précédentes, **caractérisé en ce que** l'aromatique à un ou plusieurs noyaux est choisi parmi le benzène, le toluène, les xylènes, le cumène, le diphénylméthane, les tribenzols, les tétrabenzols, les pentabenzols et les hexabenzols, le triphénylméthane, les naphtalènes substitués par alkyle, le naphtalène, les anthracènes substitués par alkyle, l'anthracène, les tétralènes substitués par alkyle et le tétralène.

11. Procédé selon l'une quelconque des revendications précédentes, **caractérisé en ce que** le benzène est transformé en cyclohexane.

12. Procédé selon l'une quelconque des revendications précédentes, **caractérisé en ce que** le benzène est transformé à une température de 80 à 180°C et le ruthénium est utilisé seul comme métal actif.
